(19) Europäisches Patentamt
European Patent Office
Office européen des brevets

(11) Publication number : **0 608 600 A1**

(12) # EUROPEAN PATENT APPLICATION

(21) Application number : **93307552.5**

(22) Date of filing : **23.09.93**

(51) Int. Cl.[5] : **A61K 7/00,** A61K 9/127

(30) Priority : **25.09.92 GB 9220268**

(43) Date of publication of application :
**03.08.94 Bulletin 94/31**

(84) Designated Contracting States :
**AT BE CH DE DK ES FR GB GR IE IT LI NL PT SE**

(71) Applicant : **UNILEVER PLC**
**Unilever House**
**Blackfriars**
**London EC4P 4BQ (GB)**
(84) **GB IE**

(71) Applicant : **UNILEVER N.V.**
**Weena 455**
**NL-3013 AL Rotterdam (NL)**
(84) **BE CH DE DK ES FR GR IT LI NL PT SE AT**

(72) Inventor : **Parrott, David Terence**
**Flat 7,**
**8 Caroline Place**
**Oxton, Birkenhead L43 1CR (GB)**
Inventor : **Turner, Jane Elizabeth**
**12 Dovedale Road,**
**Hoylake**
**Wirral, Merseyside L47 3AW (GB)**

(74) Representative : **Roscoe, Brian Corrie et al**
**UNILEVER PLC**
**Patents Division**
**Colworth House**
**Sharnbrook**
**Bedford MK44 1LQ (GB)**

(54) **Cosmetic composition.**

(57) A smectic mesophase formed in vitro comprising two or more lipids wherein the lipids together form a smectic mesophase having a repeat distance (d/o) of greater than 90Å, the individual lipid components having a d/o of less than 70Å, and its use within a cosmetic composition suitable for topical application to skin, hair or nails.

EP 0 608 600 A1

## DEFINITION OF THE INVENTION

### Field of the Invention

The invention relates to a smectic mesophase comprising two or more lipids formed in vitro and its use within a cosmetic composition suitable for topical application to skin, hair or nails.

### Background to the Invention

The vital barrier properties of the stratum corneum are thought to be mostly attributable to the inter-cellular lipids (P M Elias (1983) J Invest Dermatol 80, 44) and the structure adopted by these lipids has been the subject of intense investigation (White S H et al (1988) Biochemistry 27, 3725; Hou S Y E et al (1991) J Invest Dermatol 96, 215; Bouwstra J A et al (1991) J Controlled Release 15, 209; Garson et al (1991) J Invest Dermatol 96, 43). However these investigations have each proposed different structures. There is still therefore much ambiguity.

Our investigations have confirmed that a lamellar spacing of 131Å exists which relates to a lamellar spacing approximately twice that expected for a simple layered structure. The structure formed by the intercellular lipids has been found to be a unique smectic mesophase. Furthermore, we have surprisingly discovered that this structure can be successfully reproduced in vitro. Thus allowing cosmetic compositions to be formulated which contain lipid in the same structure as present in vivo for effective replenishment of this lipid structure known to be depleted in for example dry skin conditions.

## SUMMARY OF THE INVENTION

A smectic mesophase formed in vitro comprising two or more lipids wherein the lipids together form a smectic mesophase having a repeat distance (d/o) of greater than 90Å, the individual lipid components having a d/o of less than 70Å.

## DISCLOSURE OF THE INVENTION

Preferably the smectic mesophase has a d/o of from 90 to 150Å, more preferably from 90 to 140Å.
Preferably the individual lipid components have a d/o of from 25 to 70Å, more preferably from 30-60Å.

### Lipid Component of Smectic Mesophase

Preferably the lipid component of the smectic mesophase comprises:
(a) a ceramide; and
(b) a sterol having the general structure (1)

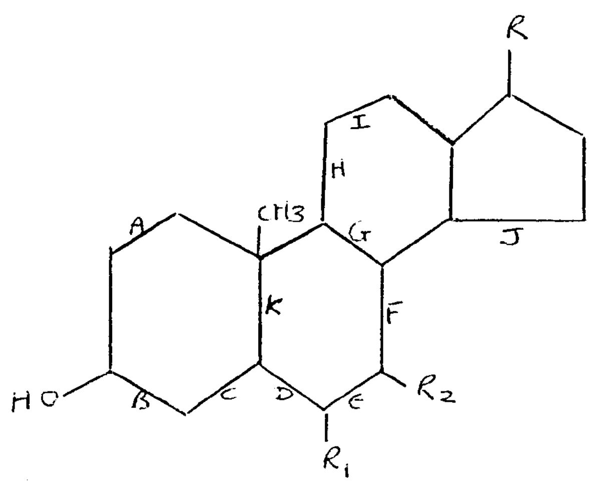

(1)

where R is a hydroxyl or a $C_{1-16}$ branched/unbranched saturated/unsaturated alkyl chain.

$R_1$ and $R_2$ individually represent hydrogen or a carbonyl.
A to K represent the bond between specified carbon atoms, these may be saturated or unsaturated.
Wherein the hydroxyl group on carbon 3 is in the β configuration.

## The Sterol Component

R is preferably a $C_{1-16}$ alkyl chain, more preferably a $C_{1-10}$ alkyl chain, most preferably a $C_{5-10}$ alkyl chain.

Preferably sterols having the general structure (1) are selected from cholesterol, pro-vitamin $D_3$ (7-dehydrocholesterol), campesterol, stigmastanol, stigmasterol, 5-dihydrocholesterol, α-spinasterol, palysterol, clionasterol, γ-sitosterol, stigmasten-3β-ol, sargasterol, avenasterol, ergostanol, β-sitosterol, corbisterol, chondrillasterol, poriferasterol, haliclonaseterol, neospongosterol, fucosterol, aptostanol, ergostadien-3β-ol, pro-vitamin $D_2$ (ergosterol), 22-dihydroergosterol, brassicasterol, 24-methylenecholesterol, 5-dihydroergosterol, dehydroergosterol, 14-dehydroergosterol, 24-dehydroergosterol, fungisterol, cholestanol, coprostanol, zymosterol, 7-ketocholesterol, lathosterol, 22-dehydrocholesterol, β-sitosterol, cholestatrien-3β-ol, coprostonal, cholestanol, ergosterol, 24-dehydrocholest-adione-3β-ol, dihydrositosterol, stigmastan-3β-ol, cholestan-3β-ol, cholestan-3β-ol-6-one , di-hydroergosterol, brassicasterol, 24-methylenecholesterol, 5-dihydroergosterol, episterol, acosterol, fecosterol, 14-dehydroergosterol, dehydroergosterol, and mixtures thereof.

More preferably the sterol is selected from cholesterol, 7-dehydrocholesterol, 5-dihydrocholesterol capesterol, ergosterol, stigmastanol, stigmasterol, and mixtures thereof.

Even more preferably the sterol is cholesterol.

## The Ceramide Component

The ceramide component is preferably selected from ceramides having the general structure (2),

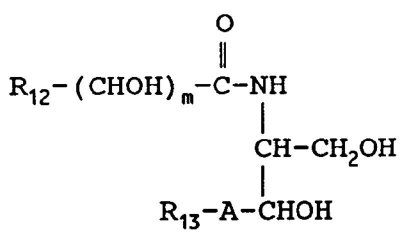

(2)

where A represents $-CH_2$ or -CH=CH- or -CHOQ-.

$R_{12}$ represents a linear or branched, saturated or unsaturated, hydroxylated or non-hydroxylated aliphatic hydrocarbon group having from 8 to 28 carbon atoms.

$R_{13}$ represents a linear or branched, saturated or unsaturated, hydroxylated or non-hydroxylated aliphatic hydrocarbon having from 8 to 28 carbon atoms.
m is 0 or 1
Q represents H or a residue of a $C_{14}$ to $C_{22}$ fatty acid having the structure (3)

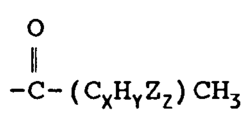

(3)

Z is -OH or an epoxy oxygen
x is an integer of from 12 to 20
y is an integer of from 20 to 40
z is 0 or an integer of from 1 to 4.

More preferably the ceramide component is a ceramide having the general structure (2) selected from;
Ceramide 2 having the structure (4)

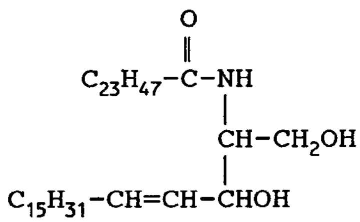

(4)

Ceramide 3 having the structure (5)

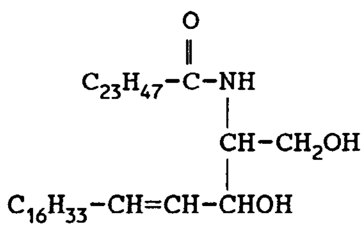

(5)

Ceramide 4 having the structure (6)

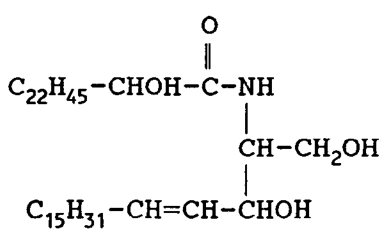

(6)

Ceramide 5 having the structure (7)

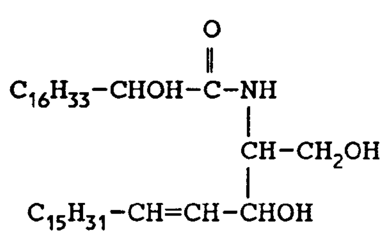

(7)

Ceramide 6(II) having the structure (8)

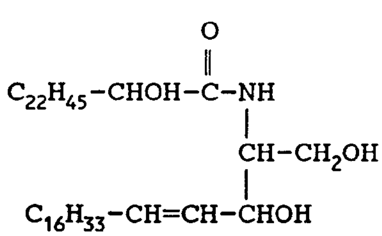

(8)

Even more preferably the ceramide component is a ceramide having the general structure (2) selected from ceramide 2 (4), ceramide 4 (6) and mixtures thereof.

Ceramides having the general structure (2) are naturally occurring and can either be synthesised or isolated from a suitable animal or plant source.

Particularly preferred sources of naturally-occurring ceramides are pig skin or neural tissue.

Since skin contains both sterol and ceramides, it is possible to form the smectic mesophase in vitro from extracted skin lipids.

Preferably the lipid components

(a) ceramide; and

(b) sterol having the general structure (1)

are present within the smectic mesophase such that the mole ratio of (a):(b) is 1:0.05 to 1:1.

More preferably the mole ratio of (a):(b) is 1:0.25 to 1:1.

Even more preferably the mole ratio of (a):(b) is 1:1.

## METHOD OF FORMING THE SMECTIC MESOPHASE

The smectic mesophase may be formed using one of two methods.

### Method One

(i) heat lipid components until melted (≧90°C)
(ii) mix components
(iii) cool to room temperature

### Method Two

(ia) dissolve lipids in suitable solvent, for example acetone, chloroform:methanol, methanol, ethanol.
(iia) mix
(iiia) evaporate solvent

## DISCLOSURE OF THE COMPOSITION

The composition according to the invention comprises in its simplest form

(a) a smectic mesophase formed in vitro comprising two or more lipids wherein the lipids together form a smectic mesophase having a repeat distance (d/o) of greater than 90Å, the individual lipid components having a d/o of less than 70Å .

(b) a cosmetically acceptable vehicle for the lipid containing smectic mesophase.

The amount of the lipid formed into the smectic mesophase present in the composition according to the invention is from 0.00001 to 50%, preferably from 0.001 to 20% and most preferably from 0.1 to 10% by weight.

The composition according to the invention may additionally comprise a lipid component which does not form part of the smectic mesophase. Preferably this additional lipid component is selected from ceramide, including ceramides having the general structure (7), pseudoceramides including pseudoceramides having the general structure (9), sterols and mixtures thereof.

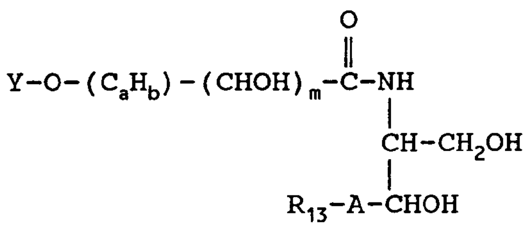

(7)

Where A represents $-CH_2$, -CH=CH- or -CHOH-,

$R_{13}$ represents a linear or branched, saturated or unsaturated, hydroxylated or non-hydroxylated aliphatic hydrocarbon having from 8 to 28 carbon atoms.

a is an integer of from 7 to 49

b is an integer of from 10 to 98

m is 0 or 1

Y represents H or a residue of a $C_{14}$ to $C_{22}$ fatty acid having the structure (8)

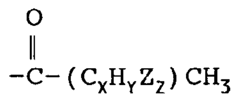 (8)

where

Z is -OH or an epoxy oxygen
x is an integer of from 12 to 20
y is an integer of from 20 to 40
z is 0 or an integer of from 1 to 4.

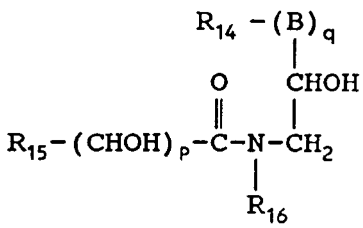 (9)

B represents $-OCH_2-$ or -CHOH-
$R_{14}$ represents a linear or branched, saturated or unsaturated, hydroxylated or non-hydroxylated aliphatic hydrocarbon group having from 8 to 28 carbon atoms.
$R_{15}$ represents a linear or branched, saturated or unsaturated, hydroxylated or non-hydroxylated aliphatic hydrocarbon group having from 8 to 49 carbon atoms or the sub-group Y-O-$(c_aH_b)$-
$R_{16}$ represents H, or a sub-group $-(CH_2)_e$, where e is an integer of from 1 to 6, or a sub-group having the structure (5)

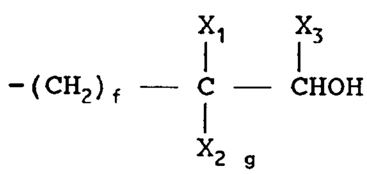 (5)

where $X_1$, $X_2$ and $X_3$ each individually represent H, a $C_{1-15}$ alkyl or a $C_{1-5}$ hydroxyalkyl;
Y represents H or a residue of a $C_{14-22}$ fatty acid having the structure

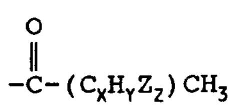 (8)

a is an integer of from 7 to 49
b is an integer of from 10 to 98
x is an integer of from 12 to 20
y is an integer of from 20 to 40
z is 0 or an integer of from 1 to 4
f is 0 or an integer of from 1 to 4
g is 0 or 1
p is 0 or 1
q is 0 or 1
where Z is OH or an epoxy oxygen.

The composition according to the invention may be used for topical application to the skin, hair or nails for improved condition. In particular the composition may be used for topical application to the skin to prevent/alleviate dry skin conditions.

The Cosmetically Acceptable Vehicle

The composition according to the invention also comprises a cosmetically acceptable vehicle to act as a dilutant, dispersant or carrier for the lipid containing smectic mesophase in the composition, so as to facilitate its distribution when the composition is applied to the skin and/or hair.

Vehicles other than water can include liquid or solid emollients, solvents, humectants, thickeners and powders. Examples of each of these types of vehicle, which can be used singly or as mixtures of one or more vehicles, are as follows:

Emollients, such as stearyl alcohol, glyceryl monoricinoleate, glyceryl monostearate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, din-butyl sebacate, isopropyl myristate, isopropyl palmitate, isopropyl stearate, butyl stearate, polyethylene glycol, triethylene glycol, lanolin, cocoa butter, corn oil, cotton seed oil, tallow, lard, olive oil, palm kernel oil, rapeseed oil, safflower seed oil, evening primrose oil, soybean oil, sunflower seed oil, avocado oil, olive oil, sesame seed oil, coconut oil, arachis oil, castor oil, acetylated lanolin alcohols, petroleum jelly, mineral oil, butyl myristate, isostearic acid, palmitatic acid, isopropyl linoleate, lauryl lactate, myristyl lactate, decyl oleate, myristyl myristate;

Propellants, such as air, propane, butane, isobutane, dimethyl ether, carbon dioxide, nitrous oxide;

Solvents, such as ethyl alcohol, methylene chloride, isopropanol, acetone, ethylene glycol monoethyl ether, diethylene glycol monobutyl ether, diethylene glycol monoethyl ether, dimethyl sulphoxide, dimethyl formamide, tetrahydrofuran;

Powders, such as chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

The cosmetically acceptable vehicle will usually form from 10 to 99.9%, Preferably from 50 to 99% by weight of the emulsion, and can, in the absence of other cosmetic adjuncts, form the balance of the composition.

OPTIONAL SKIN BENEFIT MATERIALS AND COSMETIC ADJUNTS

A particularly convenient form of the composition according to the invention is an emulsion, in which case an oil or oily material will normally be present, together with an emulsifier to provide either a water-in-oil emulsion or an oil-in-water emulsion, depending largely on the average hydrophilic-lyophilic balance (hlb) of the emulsifier employed.

Oil or oily material

The composition according to the invention can optionally comprise one or more oils or other materials having the properties of an oil.

Examples of suitable oils include mineral oil and vegetable oils, and oil materials, such as those already proposed herein as emollients. Other oils or oily materials include silicone oils, both volatile and non-volatile, such as polydimethyl siloxanes.

The oil or oily material, when present for the purposes for forming an emulsion, will normally form up to 90%, preferably from 10 to 80% by volume of the composition.

Emulsifier

The composition according to the invention can also optionally comprise one or more emulsifiers the choice of which will normally determine whether a water-in-oil or and oil-in-water emulsion is formed.

When a water-in-oil emulsion is required, the chosen emulsifier or emulsifiers should normally have an average HLB value of from 1 to 6. When an oil-in-water emulsion is required, a chosen emulsifier or emulsifiers should have an average HLB value of >6.

Examples of suitable emulsifiers are set below in Table 1 in which the chemical name of the emulsifiers is given together with an example of a trade name as commercially available, and the average HLB value.

Table 1

| Chemical Name of Emulsifier | Trade Name | HLB Value |
|---|---|---|
| Sorbitan trioleate | Arlacel 85 | 1.8 |
| Sorbitan tristearate | Span 65 | 2.1 |
| Glycerol monooleate | Aldo MD | 2.7 |
| Glycerol monostearate | Atmul 84S | 2.8 |
| Glycerol monolaurate | Aldo MC | 3.3 |
| Sorbitan sesquioleate | Arlacel 83 | 3.7 |
| Sorbitan monooleate | Arlacel 80 | 4.3 |
| Sorbitan monostearate | Arlacel 60 | 4.7 |
| Poloxyethylene (2) stearyl ether | Brij 72 | 4.9 |
| Poloxyethylene sorbitol beeswax derivative | G-1702 | 5 |
| PEG 200 dilaurate | Emerest 2622 | 6.3 |
| Sorbitan monopalmitate | Arlacel 40 | 6.7 |
| Polyoxyethylene (3.5) nonyl phenol | Emulgen 903 | 7.8 |
| PEG 200 monostearate | Tegester PEG 200 MS | 8.5 |
| Sorbitan monolaurate | Arlacel 200 | 8.6 |
| PEG 400 dioleate | Tegester PEG 400-DO | 8.8 |

| | | |
|---|---|---|
| Polyoxyethylene (5) monostearate | Ethofat 60-16 | 9.0 |
| Polyoxyethylene (4) sorbitan monostearate | Tween 61 | 9.6 |
| Polyoxyethylene (4) lauryl ether | Brij 30 | 9.7 |
| Polyoxyethylene (5) sorbitan monooleate | Tween 81 | 10.0 |
| PEG 300 monooleate | Neutronyx 834 | 10.4 |
| Polyoxyethylene (20) sorbitan tristearate | Tween 65 | 10.5 |
| Polyoxyethylene (20) sorbitan trioleate | Tween 85 | 11.0 |
| Polyoxyethylene (8) monostearate | Myrj 45 | 11.1 |
| PEG 400 monooleate | Emerest 2646 | 11.7 |
| PEG 400 monostearate | Tegester PEG 400 | 11.9 |
| Polyoxyethylene 10 monooleate | Ethofat 0/20 | 12.2 |
| Polyoxyethylene (10) stearyl ether | Brij 76 | 12.4 |
| Polyoxyethylene (10) cetyl ether | Brij 56 | 12.9 |
| Polyoxyethylene (9.3) octyl phenol | Triton X-100 | 13.0 |
| Polyoxyethylene (4) sorbitan monolaurate | Tween 21 | 13.3 |
| PEG 600 monooleate | Emerest 2660 | 13.7 |
| PEG 1000 dilaurate | Kessco | 13.9 |
| Polyoxyethylene sorbitol lanolin derivative | G-1441 | 14.0 |
| Polyoxyethylene (12) lauryl ether | Ethosperse LA-12 | 14.4 |
| PEG 1500 dioleate | Pegosperse 1500 | 14.6 |
| Polyoxyethylene (14) laurate | Arosurf HFL-714 | 14.8 |

| | | |
|---|---|---|
| Polyoxyethylene (20) sorbitan monostearate | Tween | 14.9 |
| Polyoxyethylene 20 sorbitan monooleate | Tween 80 | 15.0 |
| Polyoxyethylene (20) stearyl ether | Brij 78 | 15.3 |
| Polyoxyethylene (20) sorbitan monopalmitate | Tween 40 | 15.6 |
| Polyoxyethylene (20) cetyl ether | Brij 58 | 15.7 |
| Polyoxyethylene (25) oxypropylene monostearate | G-2162 | 16.0 |
| Polyoxyethylene (20) sorbitol monolaurate | Tween 20 | 16.7 |
| Polyoxyethylene (23) lauryl ether | Brij 35 | 16.9 |
| Polyoxyethylene (50) monostearate | Myrj 53 | 17.9 |
| PEG 4000 monostearate | Pegosperse 4000 MS | 18.7 |

The foregoing list of emulsifiers is not intended to be limiting and merely exemplifies selected emulsifiers which are suitable for use in accordance with the invention.

It is to be understood that two or more emulsifiers can be employed if desired.

The amount of emulsifier or mixtures thereof, to be incorporated in the composition of the invention, when appropriate is from 1 to 50%, preferably from 2 to 20% and most preferably from 2 to 10% by weight of the composition.

Water

The composition of the invention can also comprise water, usually up to 98%, preferably from 5 to 80% by volume.

Silicone Surfactant

The composition of the invention can also optionally comprise a high molecular weight silicone surfactant which can also act as an emulsifier, in place of or in addition to the optional emulsifier(s) already mentioned.

The silicone surfactant is a high molecular weight polymer of dimethyl polysiloxane with polyoxyethylene and/or polyoxypropylene side chains having a molecular weight of from 10,000 to 50,000 and having the structure:

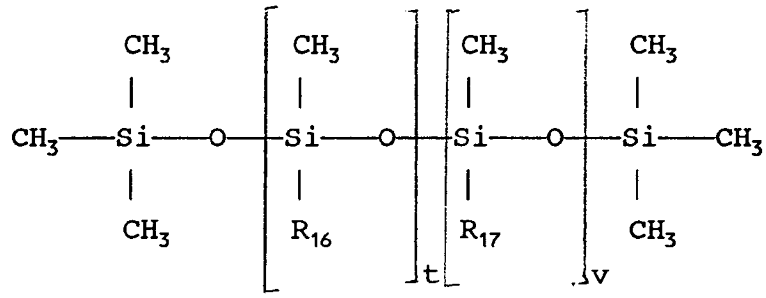

where the groups $R_{16}$ and $R_{17}$ are each chosen from -H, $C_{1-18}$ alkyl and

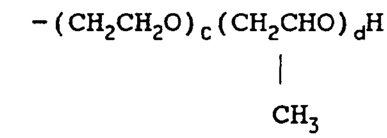

c has a value of from 9 to 115
d has a value of from 0 to 50
t has a value of from 133 to 673
v has a value of from 25 to 0.25.

Preferably, the dimethyl polysiloxane polymer is one in which:
c has a value of from 10 to 114
d has a value of from 0 to 49
t has a value of from 388 to 402
v has a value of from 15 to 0.75.

One of groups $R_{16}$ and $R_{17}$ being lauryl, and the other having a molecular weight of from 1000 to 5000.

A particularly preferred dimethyl polysiloxane polymer is one in which:
c has the value 14
d has the value 13
t has the value 249
v has the value 1.25

The dimethyl polysiloxane polymer is conveniently provided as a dispersion in a volatile siloxane, the dispersion comprising, for example, from 1 to 20% by volume of the polymer and from 80 to 99% by volume of the volatile siloxane. Ideally, the dispersion consists of a 10% by volume of the polymer dispersed in the volatile siloxane.

Examples of the volatile siloxanes in which the polysiloxane polymer can be dispersed include polydimethyl siloxane (pentamer and/or hexamer).

A particularly preferred silicone surfactant is cyclomethicone and dimethicone copolyol, such as DC 3225C formulation aid available from DOW CORNING. Another is laurylmethicone copolyol, such as DC Q2-5200, also available from Dow Corning.

The amount of silicone surfactant, when present in the composition will normally be up to 25%, preferably from 0.5 to 15% by weight of the emulsion.

Other Cosmetic Adjuncts

Examples of conventional adjuncts which can optionally be employed include preservatives, such as parahydroxy benzoate esters; antioxidants, such butyl hydroxy toluene; humectants, such as glycerol, sorbitol, 2-pyrrolidone-5-carboxylate, dibutylphthalate, gelatin, polyethylene, glycol, preferably PEG 200-600; buffers, such as lactic acid together with a base such as triethanolamine or sodium hydroxide; surfactants, such as glycerol ethers and other ceramides of synthetic, animal or plant origin including ceramide one; phospholipids; waxes, such as beeswax, ozokerite wax, paraffin wax, plant extracts, such as aloe vera, cornflower, witch hazel, elderflower, cucumber; additional sterols, particularly cholesterol; thickeners; activity enhancers; colourants; perfumes; and sunscreen materials such as ultrafine titanium dioxide and organic sunscreens such as p-aminobenzoic acid and esters thereof, ethylhexyl p-methoxycinnamate, 2-ethoxyethyl p-methoxycinnamate

and butyl methoxydibenzoylmethane, and mixtures thereof.

Cosmetic adjuncts can form the balance of the composition.

Use of the Composition

The composition according to the invention is intended primarily as a product for topical application to human skin, especially as an agent for reducing the permeability to water of the skin, particularly when the skin is dry or damaged, in order to reduce moisture loss and generally to enhance the quality and flexibility of skin. The composition can also be applied to hair and nails.

In use, a small quantity of the composition, for example from 1 to 5 ml, is applied to exposed areas of the skin, from a suitable container or applicator and, if necessary, it is then spread over and/or rubbed into the skin using the hand or fingers or a suitable device.

PRODUCT FORM AND PACKAGING

The topical skin, hair or nail treatment composition of the invention can be formulated as a lotion having a viscosity of from 4,000 to 10,000 mPas, a fluid cream having a viscosity of from 10,000 to 20,000 mPas or a cream having a viscosity of from 20,000 to 100,000 mPas, or above. The composition can be packaged in a suitable container to suit its viscosity and intended use by the consumer.

For example, a lotion or fluid cream can be packaged in a bottle or a roll-ball applicator or a propellant-driven aerosol device or a container fitted with a pump suitable for finger operation. When the composition is a cream, it can simply be stored in a non-deformable bottle or squeeze container, such as a tube or a lidded jar.

The invention accordingly also provides a closed container containing a cosmetically acceptable composition as herein defined.

EXAMPLES

The invention is illustrated by the following examples.

Examples 1-14

Materials

Ceramide II (Sigma Type III) and ceramide IV (Sigma type IV) bovine brain ceramides and cholesterol, campesterol, stigmastanol, stigmasterol, 7-hydrocholesterol, dihydrocholesterol, 5β-cholesten-3α-ol, were obtained from the Sigma Chemical Company and were used without further purification.

Formation of the smectic mesophase

Lipids were mixed by heating beyond the liquid transition temperature, then cooled.

X-ray Diffraction

Samples were mixed and transferred to 1 mm ID Lindermann tubes two days before measurement. SAXD experiments were carried out on line 8.2 at Daresbury. Camera lengths of 1.5 or 1.7 m, a wavelength of 1.500 nm and a quadrant detector system were used. Temperature was varied using a modified Linkam TMS 600 hot-stage and controlled to 0.2°C with a TMS central processor. All samples were exposed for three minutes.

Examples 1-8

The effect of increasing cholesterol concentration on the small angle diffraction pattern of ceramide II was studied as follows:

| Example | Mole ratios cholesterol:ceramide II |
|---|---|
| 1 | 0:1 |
| 2 | 0.098:1 |
| 3 | 0.16:1 |
| 4 | 0.27:1 |
| 5 | 0.37:1 |
| 6 | 0.49:1 |
| 7 | 0.85:1 |
| 8 | 1.6:1 |

The lamellar spacing of "pure" ceramide II (example 1) was 55Å. This spacing did not change the following cooling in situ from isotropic, suggesting that the stable form was present.

Three changes in the diffraction pattern of ceramide II, occur on the addition of cholesterol in samples measured immediately after cooling from isotropic.

(i) The lamellar d/o reflection reduces from 55Å to 41Å as the cholesterol content is increased in relation to the ceramide to a mole ratio of 1.6:1.

(ii) A new peak evolves at 104Å. This is clear in Example 4 although this peak is clearly visible in Example 2 if the sample is aged over two days. This increases in intensity on further addition of cholesterol. This reflection arises from a layered structure, since d/2 and d/3 are visible, although the former is convoluted with the peak described in (i). Increasing the level of cholesterol does not change the d/o value.

(iii) Example 8 showed no evidence of the 104Å reflection, previously induced at lower cholesterol content, but only the d/o and d/2 in (i).

Figures 1-8 show the results of studies on Examples 1-8 respectively.

Examples 9-13 & Comparative Example A

The effect of various sterols on the small angle diffraction pattern of ceramide II or ceramide IV was studied as follows:

| Example | Sterol & ceramide studied in 1:1 mole ratio |
|---|---|
| 9 | 7-dehydrocholesterol & ceramide IV |
| 10 | dihydrocholesterol & ceramide II |
| 11 | campesterol & ceramide II |
| 12 | stigmastanol & ceramide IV |
| 13 | stigmasterol & cermide II |
| A | 5β-cholesten-3α-ol & ceramide II |

Results are shown in Figures 9-14 respectively.

A smectic mesophose according to the invention was formed with Examples 9-13. Comparative Example A ilustrates the requirement for the hydroxyl group located on carbon number 3 to be in the β configuration, no smectic mesophase being formed when the hydroxyl group is in the α position (as in 5β-cholesten-3α-ol).

Example 14

This example illustrates a high internal phase water-in-oil emulsion in accordance with the invention.

Initially a smectic mesophase was prepared containing ceramide 2 having the structure (4) and cholesterol in a 1:1 mole ratio.

Secondly a high internal phase water-in-oil emulsion having the following formulation was prepared:

| | % w/w |
|---|---|
| Fully Hydrogenated coconut oil | 3.9 |
| Lipid components forming smectic mesophase As detailed above | 0.1 |
| Brij 92* | 5 |
| Bentone 38 | 0.5 |
| Preservative | 0.3 |
| $MgSO_4 7H_2o$ | 0.3 |
| Butylated hydroxy toluene | 0.01 |
| Perfume | qs |
| Water | to 100 |

*Brij 92 is polyoxyethylene (2) oleyl ether

Example 15

This example also illustrates a high internal phase water-in-oil emulsion in accordance with the invention in which the formulation of Example 14 was prepared but with the following changes:

i. liquid paraffin replaced the fully hydrogenated coconut oil, and

ii. the lipid component used to form the smectic mesophase were ceramide 4 having the structure (6) in a 1:1 mole ratio.

Example 16

This example also illustrates a high internal phase water-in-oil emulsion in accordance with the invention in which the formulation of Example 14 was prepared but with the following changes:

The lipid component used to form the smectic mesophase were ceramide 4 having the structure (6), ceramide 2 having the structure (4) and cholesterol. The ratio of ceramide 4 to ceramide 2 was 1:1 and the mole ratio of total ceramide to cholesterol was 1:1.

Example 17

This example illustrates an oil-in-water cream.

Firstly a smectic mesophase was formed from a ceramide 3 having the structure (5) and 5-dihydrocholesterol in a mole ratio of 1:0.25.

Secondly an oil-in-water cream emulsion having the following formulation was prepared:

| | % w/w |
|---|---|
| Mineral oil | 4 |
| Smectic mesophase as detailed above | 0.1 |
| Brij 56* | 4 |
| Alfol 16RD* | 4 |
| Triethanolamine | 0.75 |
| Butane-1,3-diol | 3 |
| Xanthan gum | 0.3 |
| Preservative | 0.4 |
| Perfume | qs |
| Butylated hydroxy toluene | 0.01 |
| Water | to 100 |

*Brij 56 is cetyl alcohol POE (10)
Alfol 16RD is cetyl alcohol

Example 18

This example illustrates an alcoholic lotion in accordance with the invention.

Initially a smectic mesophase was formed form ceramide 2 having the structure (4) and cholesterol in a 1:0.75 mole ratio. Then a lotion was prepared having the following formulation.

| | % w/w |
|---|---|
| Smectic mesophase as detailed above | 0.2 |
| Ethanol | 40 |
| Perfume | qs |
| Butylated hydroxy toluene | 0.01 |
| Water | to 100 |

Example 19

The following composition according to the invention represents a lotion which can be used in the treatment of dry skin.

Initially a smectic mesophase was formed from ceramide 2 having the structure (4) and cholesterol in a 1:1 mole ratio. This was then incorporated in the composition as follows:

| | % w/w |
|---|---|
| Smectic mesophase as detailed above | 1.0 |
| Ceramide 1 having the structure (7) | 0.5 |
| Perfume | 0.1 |
| Hydroxyethyl cellulose | 0.4 |
| Absolute ethanol | 25 |
| p-methyl benzoate | 0.2 |
| Sterilised demineralised water | to 100 |

Example 20

The following compositions according to the invention represent lotions which can be used in the treatment of dry skin.

Initially a smectic mesophase was formed from ceramide 4 having the structure (6) and cholesterol in a 1:1 mole ratio. This was then incorporated in the composition as follows:

| | % w/w |
|---|---|
| Smectic mesophase as detailed above | 0.08 |
| Pseudoceramide having the structure (9) | 0.15 |
| Ethanol | 10 |
| Perfume | 0.5 |
| Distilled water | to 100 |

## Claims

1. A smectic mesophase formed in vitro comprising two or more lipids wherein the lipids together form a smectic mesophase having a repeat distance (d/o) of greater than 90Å, the individual lipid components having a d/o of less than 70Å.

2. A smectic mesophase according to claim 1 wherein the smectic mesophase has a d/o of from 90 to 150Å.

3. A smectic mesophase according to claim 1 & 2 wherein the smectic mesophase has a d/o of from 90 to 140Å.

4. A smectic mesophase according to any preceding claim wherein the individual lipid components have a d/o of from 25 to 70Å.

5. A smectic mesophase according to any preceding claim wherein the individual lipid components have a d/o of from 30 to 60Å.

6. A smectic mesophase according to any preceding claim comprising; (a) a ceramide; and (b) a sterol having the general structure (1):

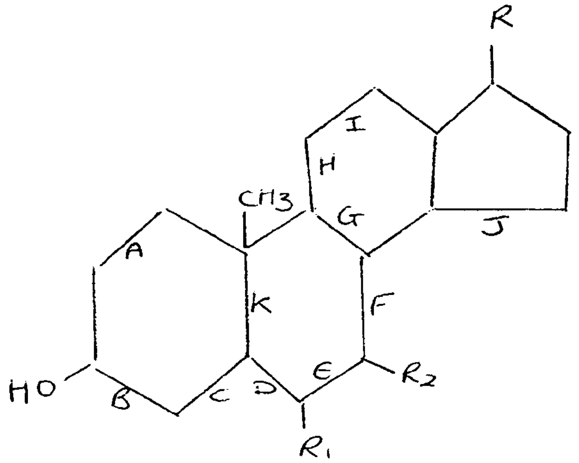

(1)

where R is a hydroxyl or a $C_{1-16}$ branched/unbranched; saturated/unsaturated alkyl chain;
$R_1$ and $R_2$ individually represent H or a carbonyl group;
A to K represent the bond between specified carbon atoms within the sterol structure, these may be saturated or unsaturated; and
wherein the hydroxyl group on carbon 3 is in the β configuration.

7. A smectic mesophase according to claim 6 wherein the ceramide has the general structure (2):

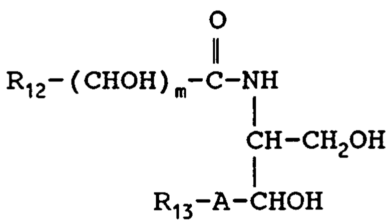

(2)

where A represents $-CH_2$ or -CH=CH- or -CHOQ-.
$R_{12}$ and $R_{13}$ each individually represents a linear or branched, saturated or unsaturated, hydroxylated or nonhydroxylated aliphatic hydrocarbon group having from 8 to 28 carbon atoms;
m is 0 or 1
q represents H or a residue of a $C_{14}$ to $C_{22}$ fatty acid having the structure (3)

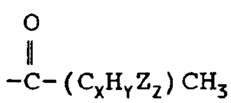

(3)

Z is -OH or an epoxy oxygen
x is an integer of from 12 to 20
y is an integer of from 20 to 40
z is 0 or an integer of from 1 to 4.

8. A smectic mesophase according to claims 6 and 7 wherein the ceramide component is selected from ceramide 2, ceramide 3, ceramide 4, ceramide 5, ceramide 6 (II) and mixtures thereof.

9. A smectic mesophase according to claims 6 to 8 wherein the ceramide component is selected from ceramide 2 and ceramide 4.

10. A smectic mesophase according to claim 6 to 9 wherein the sterol is selected from cholesterol, 7-dehydrocholesterol, campesterol, stigmastanol, stigmasterol, 5-dihydrocholesterol, ergosterol and mixtures thereof.

**11.** A smectic mesophase according to claims 6 to 10 wherein the sterol is cholesterol.

**12.** A smectic mesophase according to claims 6 to 11 wherein the mole ratio of ceramide:sterol is 1:0.05 to 1:1.

**13.** A smectic mesophase according to claims 6 to 12 wherein the mole ratio of ceramide:sterol is 1:0.25 to 1:1.

**14.** A smectic mesophase according to claims 6 to 13 wherein the mole ratio of ceramide:sterol is 1:1.

**15.** A cosmetic composition comprising
(a) a smectic mesophase as claimed by any preceding claim
(b) a cosmetically acceptable vehicle for the lipid containing smectic mesophase.

**16.** A composition according to claim 15 wherein the composition contains an effective amount of from 0.00001 to 50% by weight of lipid forming the smectic mesophase.

**17.** A composition according to claims 15 and 16 wherein the composition additionally comprises a lipid component which does not form part of the smectic mesophase.

**18.** A cosmetic composition according to claim 17 wherein the additional lipid component is selected from ceramide, pseudoceramides, sterols and mixtures thereof.

**19.** Use of a cosmetic composition according to claims 15 to 18 for topical application to skin to prevent and/or alleviate dry skin conditions.

*Fig.1.*

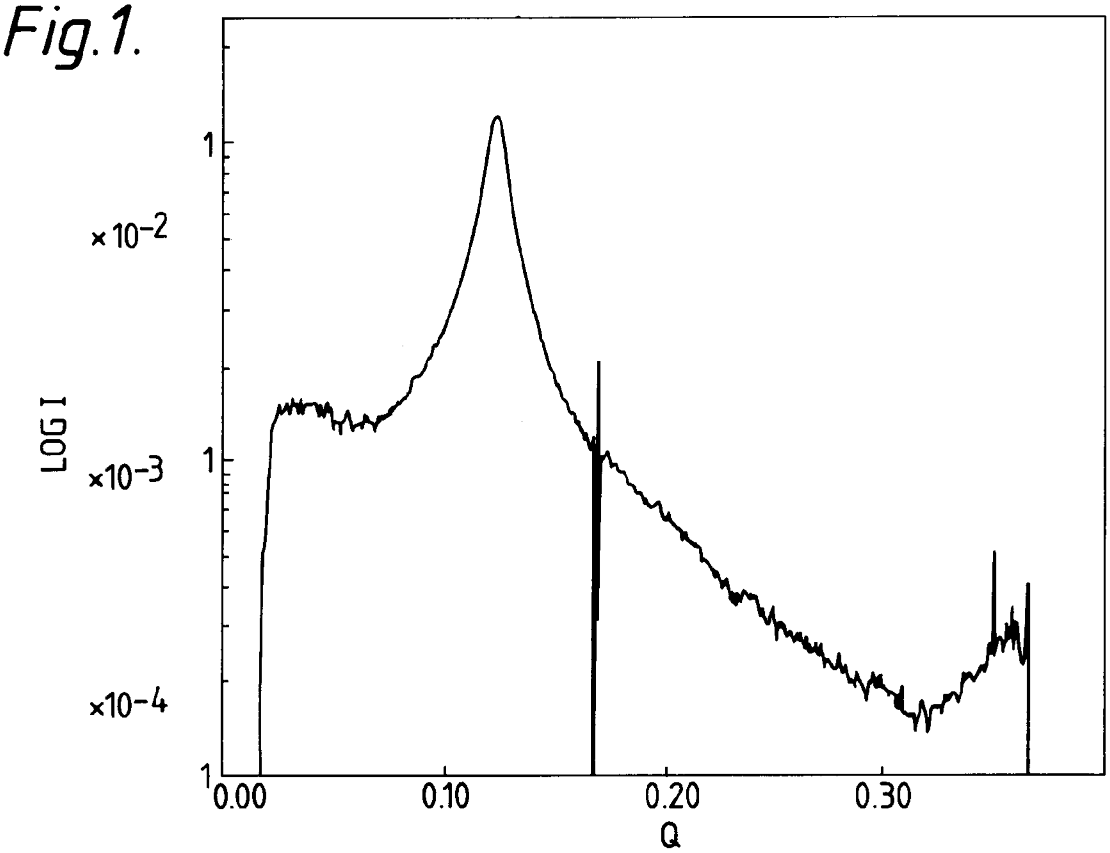
LOG I
1
×10^-2
1
×10^-3
×10^-4
1
0.00
0.10
0.20
0.30
Q

*Fig.2.*

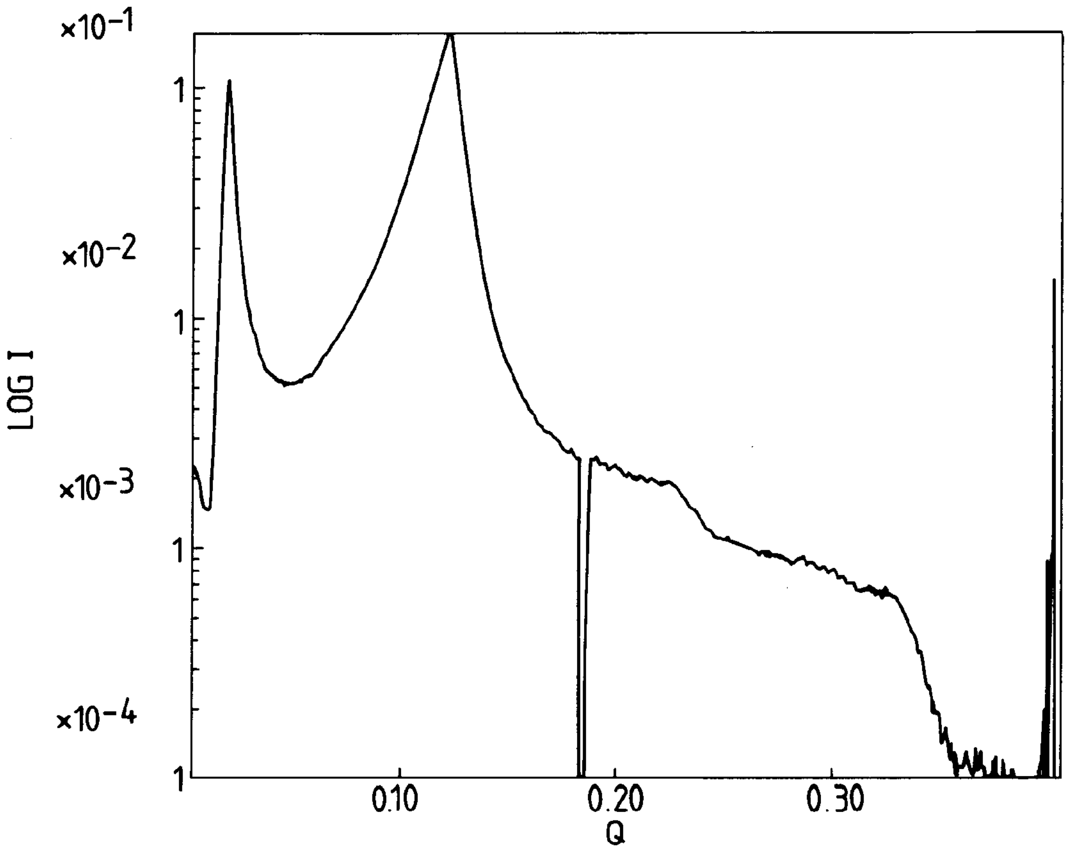
×10^-1
1
×10^-2
1
LOG I
×10^-3
1
×10^-4
1
0.10
0.20
0.30
Q

Fig. 3.

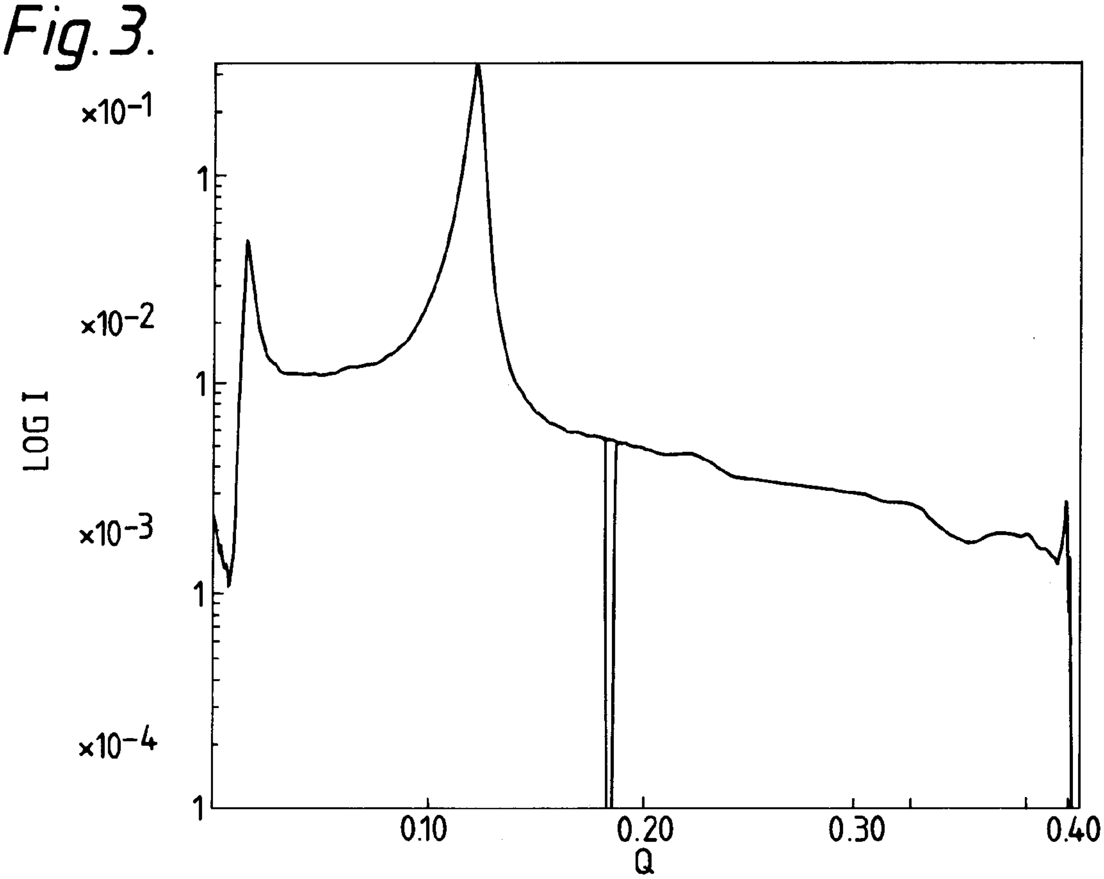
×10−1
1
×10−2
1
×10−3
1
×10−4
1
LOG I
0.10
0.20
0.30
0.40
Q

Fig. 4.

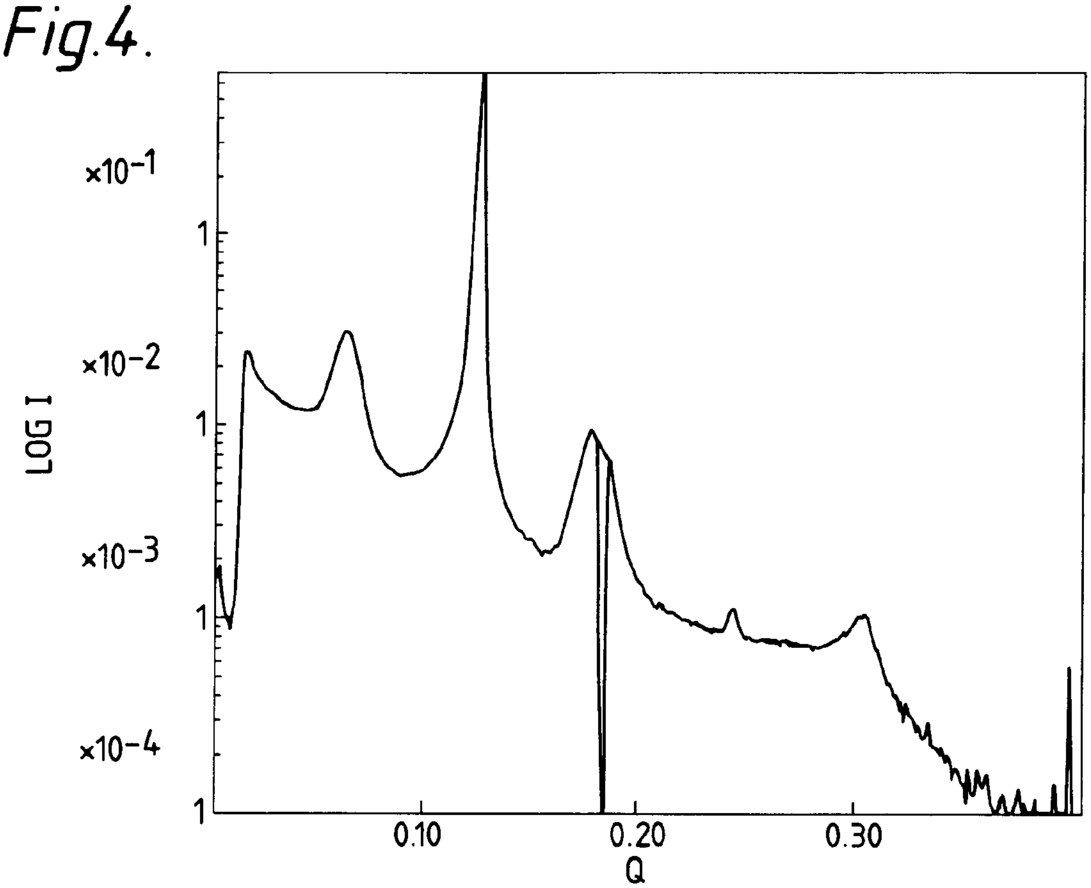
×10−1
1
×10−2
1
×10−3
1
×10−4
1
LOG I
0.10
0.20
0.30
Q

Fig.5.

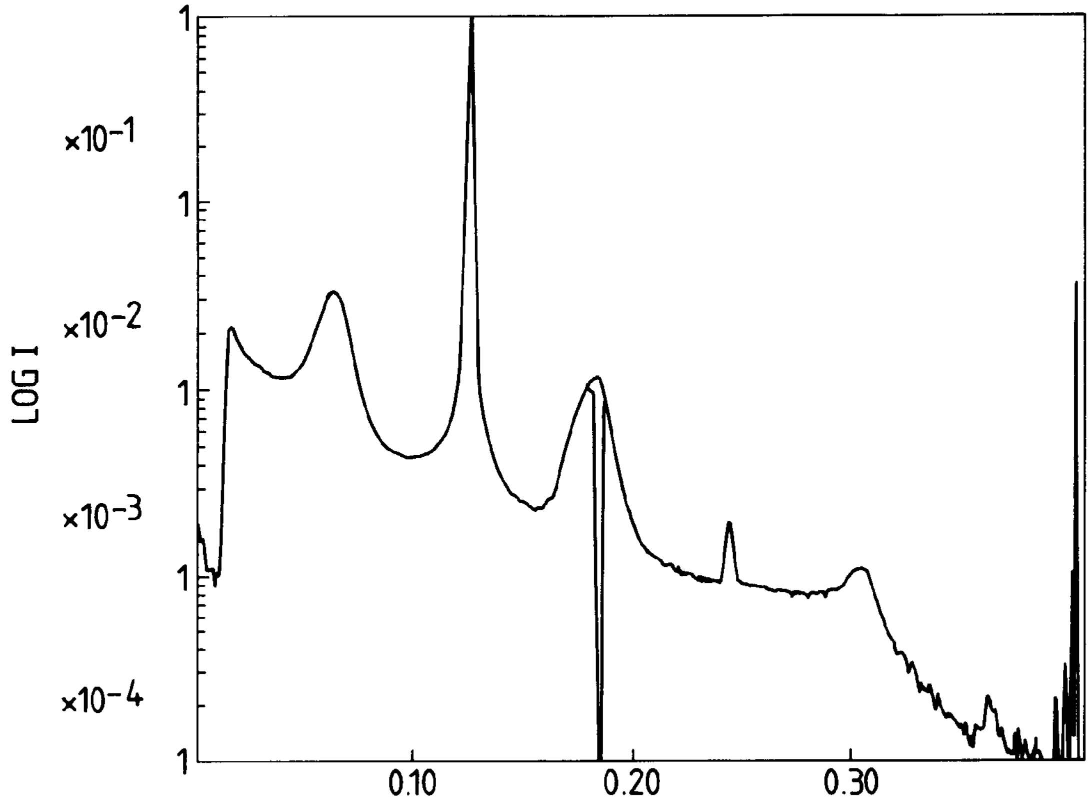
1
×10−1
1
×10−2
1
×10−3
1
×10−4
1
LOG I
0.10
0.20
0.30

Fig.6.

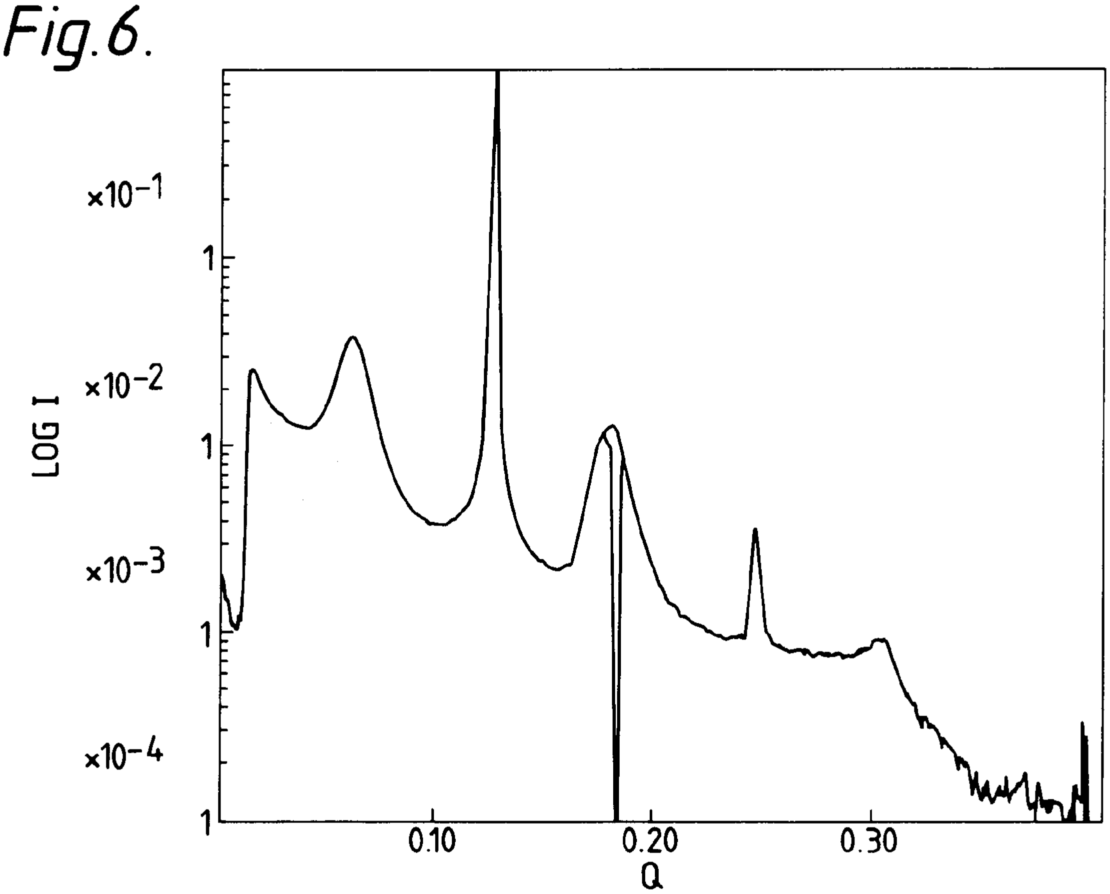
×10−1
1
×10−2
1
×10−3
1
×10−4
1
LOG I
0.10
0.20
0.30
Q

Fig. 7.

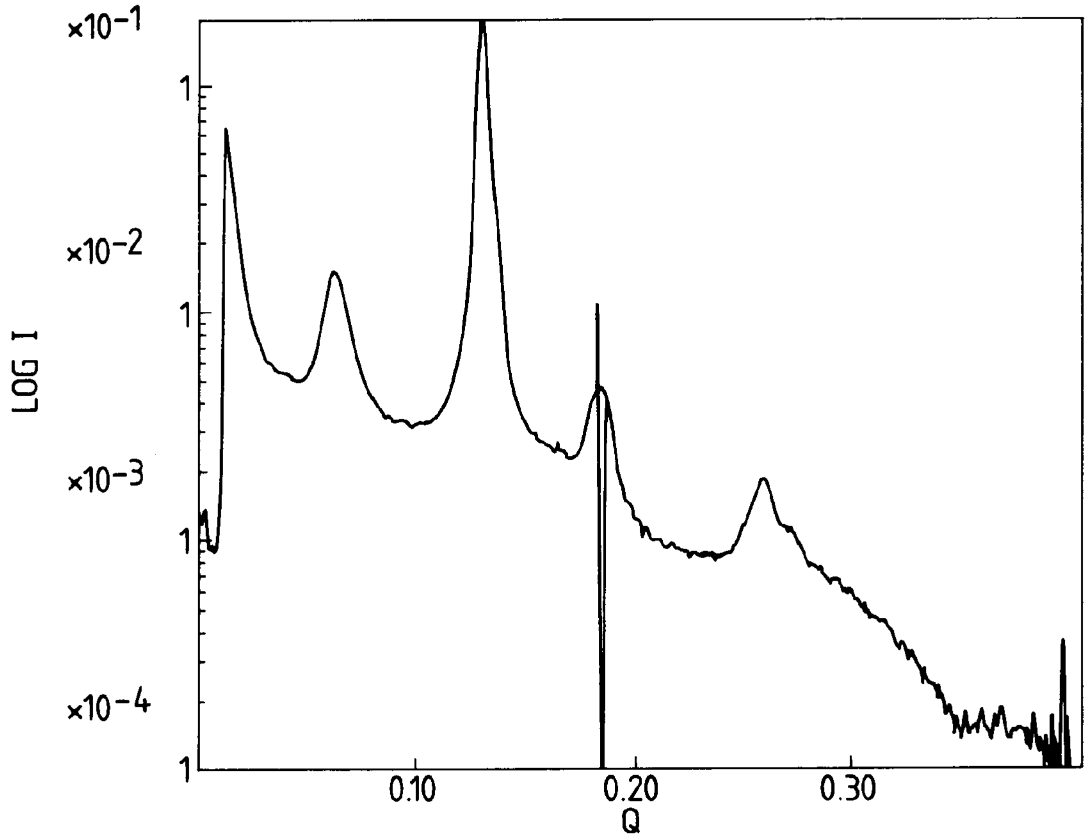
×10−1
1
×10−2
1
×10−3
1
×10−4
1
LOG I
0.10
0.20
0.30
Q

Fig. 8.

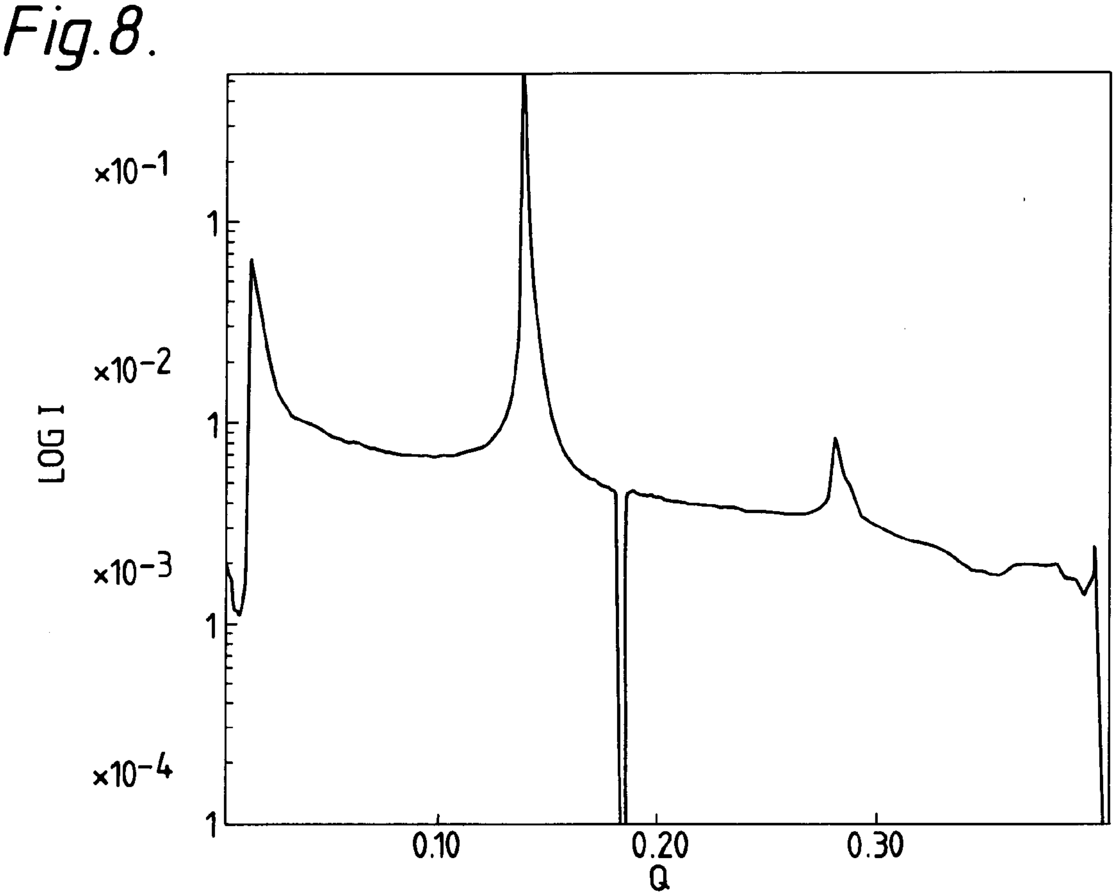
×10−1
1
×10−2
1
×10−3
1
×10−4
1
LOG I
0.10
0.20
0.30
Q

Fig. 9.

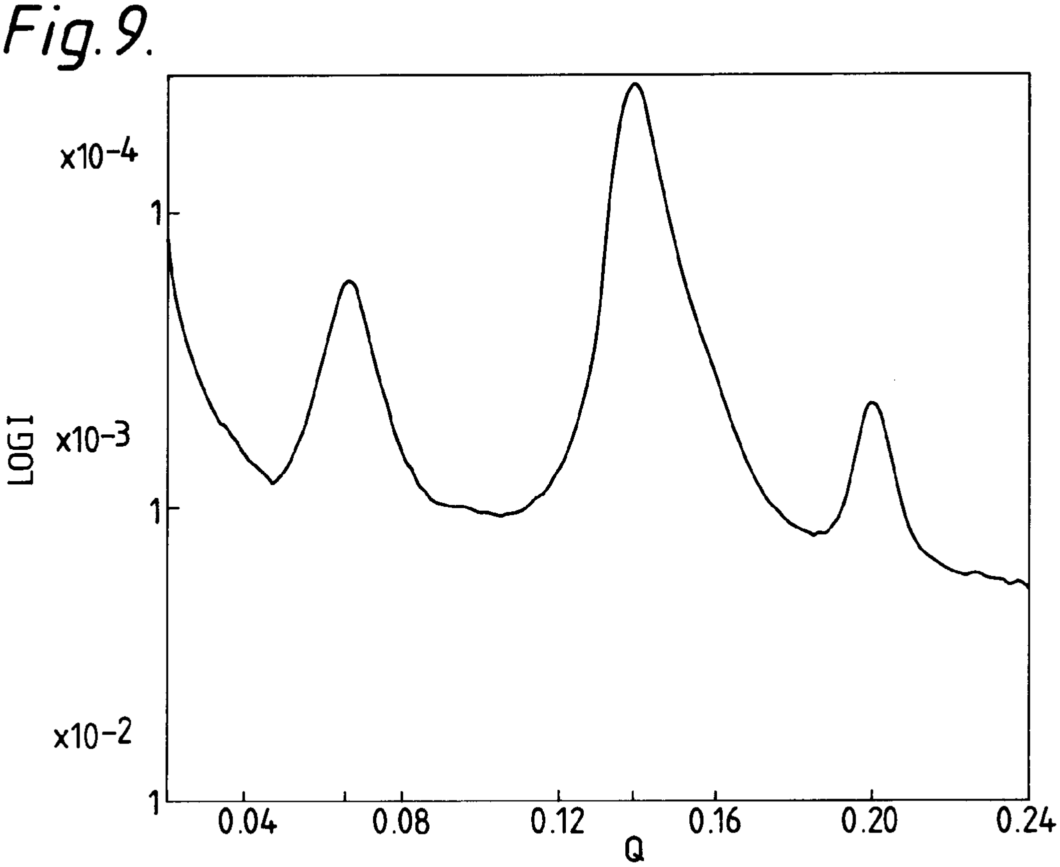
x10-4
1
LOG I
x10-3
1
x10-2
1
0.04
0.08
0.12
0.16
0.20
0.24
Q

Fig. 10.

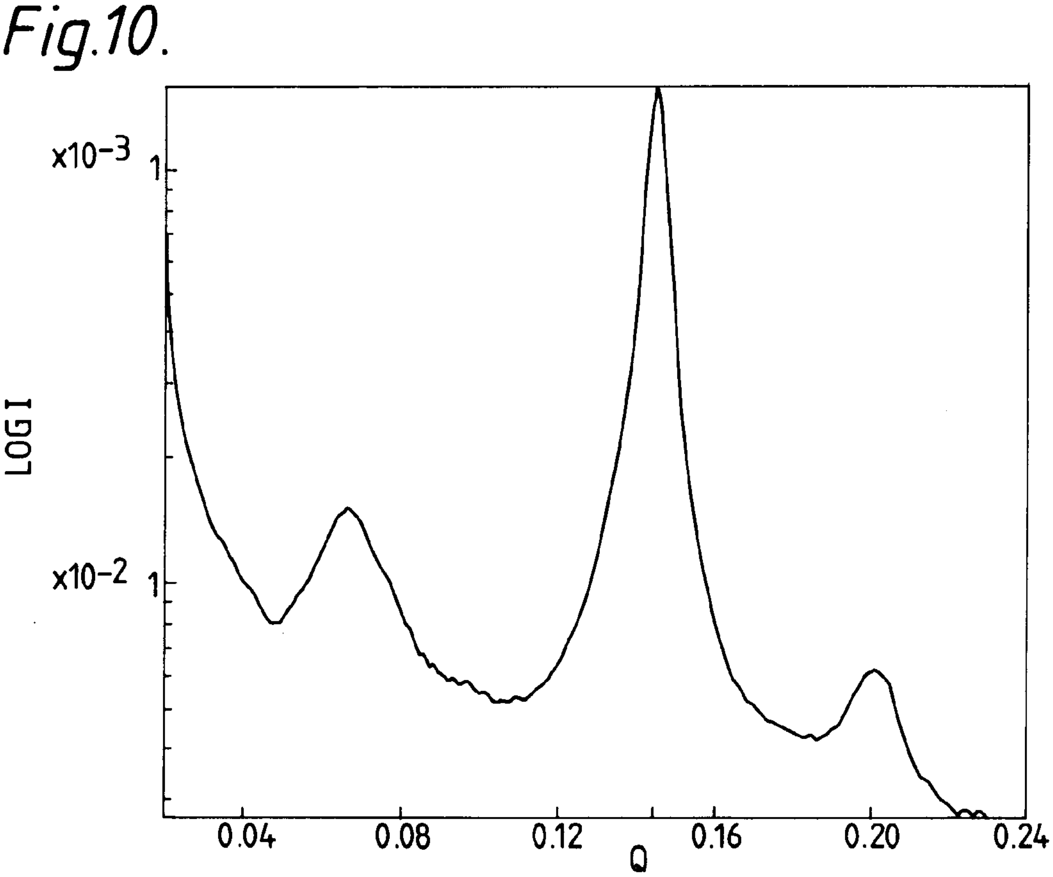
x10-3
1
LOG I
x10-2
1
0.04
0.08
0.12
0.16
0.20
0.24
Q

Fig.11.

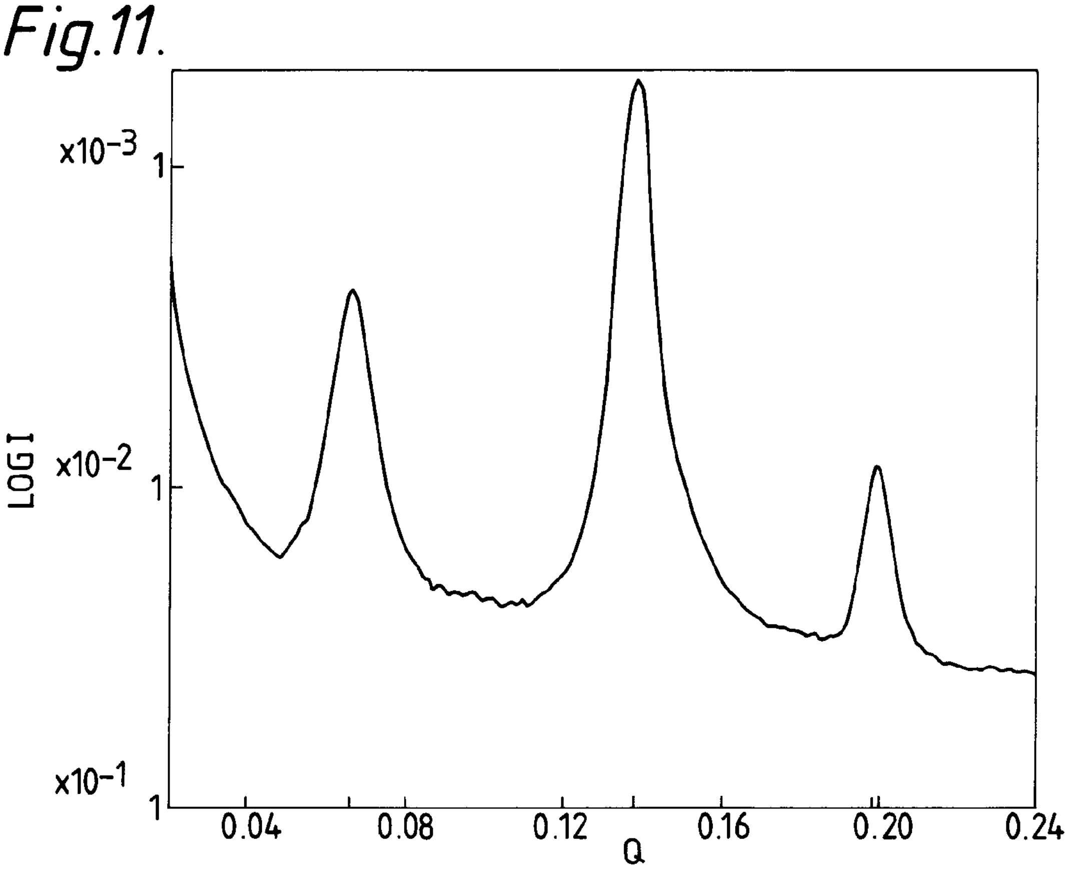
x10−3 1
x10−2 1
x10−1 1
LOG I
0.04
0.08
0.12
0.16
0.20
0.24
Q

Fig.12.

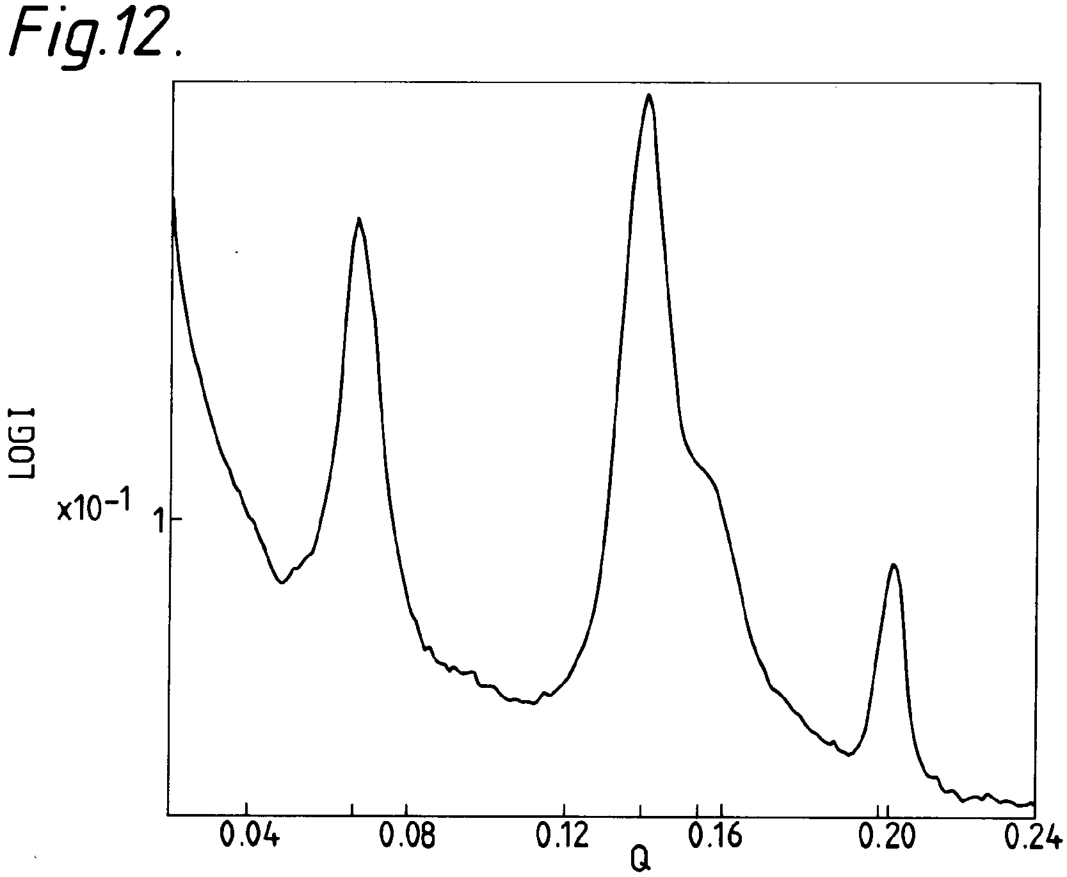
x10−1 1
LOG I
0.04
0.08
0.12
0.16
0.20
0.24
Q

Fig.13.

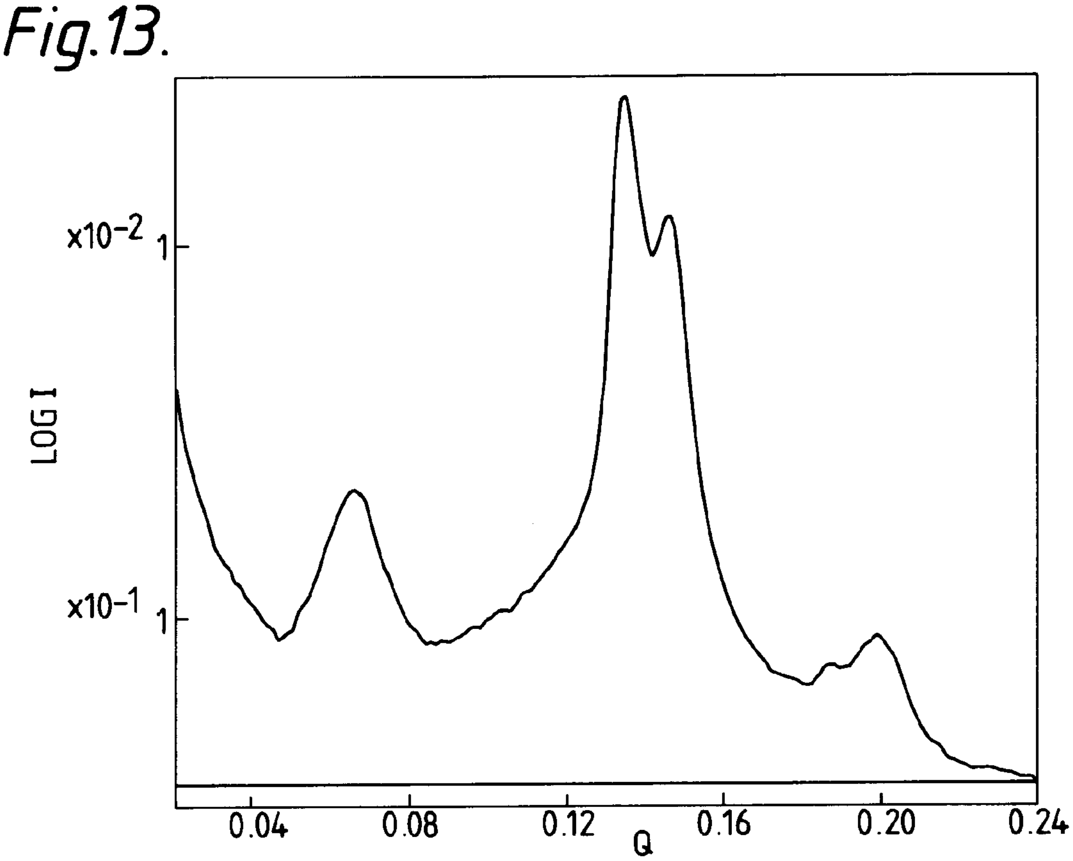
x10−2 1
x10−1 1
LOG I
0.04
0.08
0.12
0.16
0.20
0.24
Q

Fig.14.

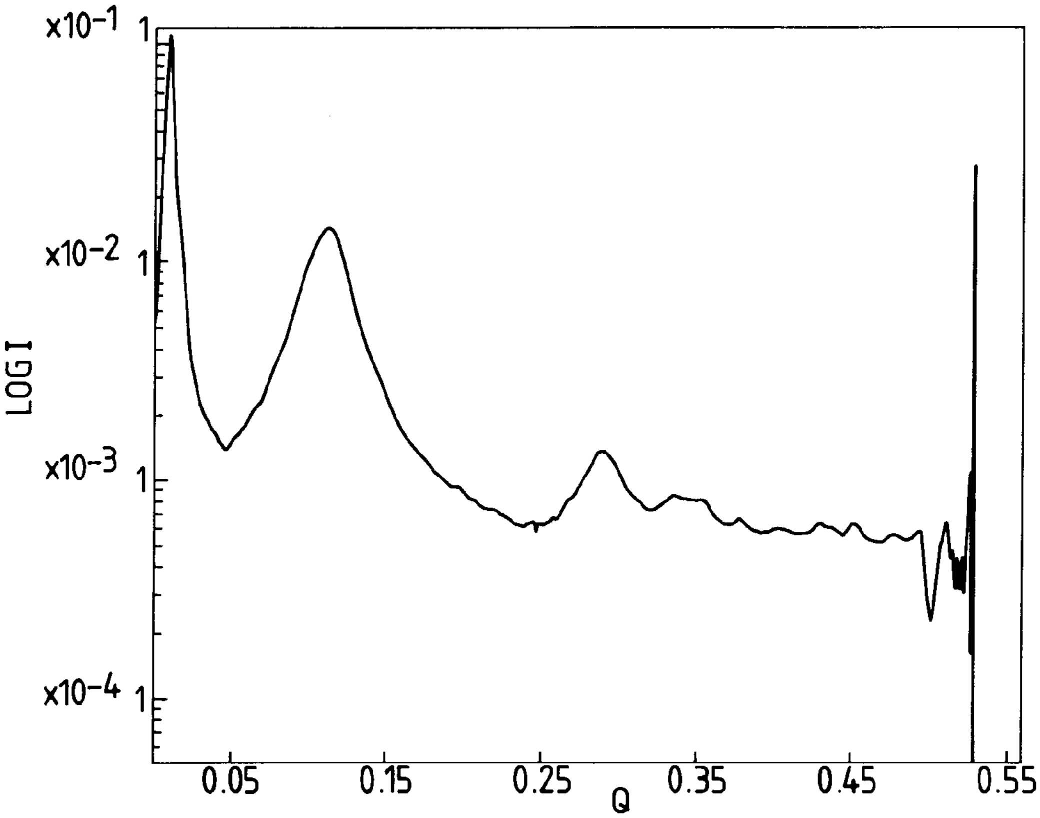
x10−1 1
x10−2 1
x10−3 1
x10−4 1
LOG I
0.05
0.15
0.25
0.35
0.45
0.55
Q

European Patent Office

# EUROPEAN SEARCH REPORT

Application Number
EP 93 30 7552

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y | EP-A-0 420 722 (L'OREAL)<br>* claims 1-16; example V * | 1-19 | A61K7/00<br>A61K9/127 |
| Y | EP-A-0 500 437 (L'OREAL)<br>* claims 1-19; examples 1,2,8 * | 1-19 | |
| A | WO-A-91 04013 (MICRO VESICULAR SYSTEMS)<br>* claims 1-18; examples 4-6 * | 1-19 | |
| P,Y | EP-A-0 556 957 (UNILEVER)<br>* claims 1-18 * | 1-19 | |

TECHNICAL FIELDS SEARCHED (Int.Cl.5)
A61K

The present search report has been drawn up for all claims

| Place of search | Date of completion of the search | Examiner |
|---|---|---|
| THE HAGUE | 21 March 1994 | Willekens, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)